# EUROPEAN PATENT APPLICATION

(11) **EP 4 772 881 A1**
(43) Date of publication of application: **08.07.2026**
(21) Application number: 24859583.7
(22) Date of filing: 22.08.2024
(51) Int. Cl.: G01N 33/531, C07K 16/44, G01N 33/53

(54) **ANTI-PEG ANTIBODY-BINDING MATERIAL AND DETECTION METHOD FOR ANTI-PEG ANTIBODY**

(30) Priority: 29.08.2023 JP 2023138749
(71) Applicant: INSTITUTE OF SCIENCE TOKYO, Tokyo 152-8550 (JP); DKS Co. Ltd., Kyoto-shi, Kyoto 600-8873 (JP)
(72) Inventor: SERIZAWA Takeshi, Tokyo 152-8550 (JP); SAWADA Toshiki, Tokyo 152-8550 (JP); NISHIURA Masahito, Kyoto-shi, Kyoto 600-8873 (JP)
(74) Representative: Hoffmann Eitle
(86) International application number: PCT/JP2024/029727
(87) International publication number: WO 2025/047550

(57) **Abstract**

Provided is an anti-PEG antibody-binding material capable of detecting an anti-PEG antibody. An anti-PEG antibody-binding material according to an embodiment includes a cellulose oligomer represented by general formula (1). In formula (1), n represents a number of 6 to 16, m represents a number of 3 or more, and A represents a divalent organic group that connects the oxygen atom bonded to the anomeric carbon of the reducing end of the cellulose oligomer and a polyethylene glycol chain and that has 1 to 24 carbon atoms and contains at least one heteroatom selected from the group consisting of a nitrogen atom, an oxygen atom, and a sulfur atom.

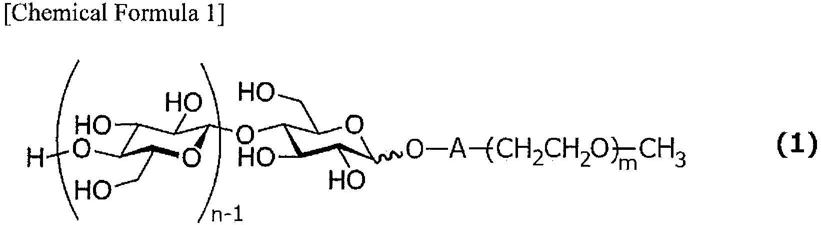

## Description

### Technical Field

The present invention relates to an anti-PEG antibody-binding material and also to a detection method for an anti-PEG antibody using the same.

### Background Art

When proteins, liposomes, micelles, and the like are surface-modified with PEG (polyethylene glycol), the modified materials have improved blood retention, and their immunogenicity is reduced. Therefore, many PEG-modified pharmaceuticals are currently in clinical use.

However, it has been revealed that PEG modification induces the secretion of antibodies against PEG (anti-PEG antibodies). The administration of a PEG-modified pharmaceutical while anti-PEG antibodies are present in the blood may cause a phenomenon in which the PEG-modified pharmaceutical is cleared from the blood (ABC phenomenon), leading to a decrease in the blood retention or the occurrence of side effects. Therefore, in order to evaluate the impact on the induction of anti-PEG antibodies and the therapeutic efficacy of PEG-modified pharmaceuticals, there is a demand for highly sensitive detection of anti-PEG antibodies present in the blood.

Anti-PEG antibodies include backbone-selective anti-PEG antibodies and methoxy-terminated PEG-selective anti-PEG antibodies. A backbone-selective anti-PEG antibody is an antibody that recognizes the oxyethylene repeating structure, which is the backbone of the PEG structure, and specifically binds to such PEG chains. A methoxy-terminated PEG-selective anti-PEG antibody is an antibody that has high selectivity for methoxy-terminated PEG, that is, it specifically binds to PEG chains terminated with a methoxy group.

As a technology relating to a biosensor containing PEG chains, for example, PTL 1 discloses a biosensor containing PEG-modified nanoparticles, in which a functional group or a functional moiety is attached to a metal (oxide) microparticle or a semiconductor microparticle via two or more PEG chains.

In addition, PTLs 2 and 3 disclose biosensors containing hydrophilic particles, each including a water-insoluble particle, a polysiloxane-containing primer layer disposed on the surface thereof, and a hydrophilic polymer layer disposed on the primer layer. The hydrophilic polymer layer is composed of PEG chains having an antibody bound thereto and PEG chains having an inactive group bonded thereto.

### Citation List

### Patent Literature

PTL 1: JP2005-180921A
PTL 2: JP2020-143964A
PTL 3: JP2021-012089A

### Summary of Invention

### Technical Problem

In the course of research aimed at detecting anti-PEG antibodies present in the blood with high sensitivity, the present inventors have found that a specific cellulose oligomer having a PEG chain specifically binds to an anti-PEG antibody.

Embodiments of the invention have been made in view of the above points, and an object thereof is to provide an anti-PEG antibody-binding material capable of binding to an anti-PEG antibody, and also a detection method for an anti-PEG antibody using the same.

### Solution to Problem

The invention includes the following embodiments.
[1] An anti-PEG antibody-binding material including a cellulose oligomer represented by general formula (1). 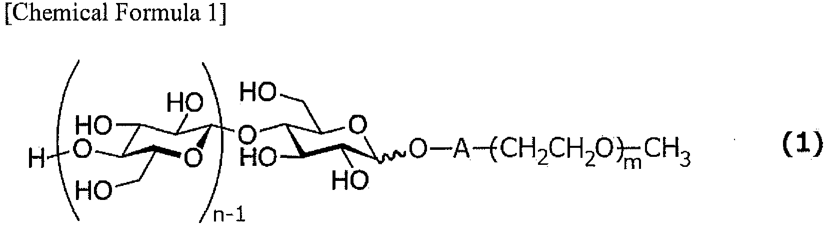 (In formula (1), n is the average degree of polymerization and represents a number of 6 to 16, m is the average degree of polymerization and represents a number of 3 or more, and A represents a divalent organic group that connects the oxygen atom bonded to the anomeric carbon of the reducing end of the cellulose oligomer and a polyethylene glycol chain and that has 1 to 24 carbon atoms and contains at least one heteroatom selected from the group consisting of a nitrogen atom, an oxygen atom, and a sulfur atom.)
[2] The anti-PEG antibody-binding material according to [1], in which A in the formula (1) represents a divalent organic group having 2 to 10 carbon atoms and containing at least one member selected from the group consisting of a triazole group, an amide group, and a thioether group.
[3] The anti-PEG antibody-binding material according to [1] or [2], in which A in the formula (1) represents 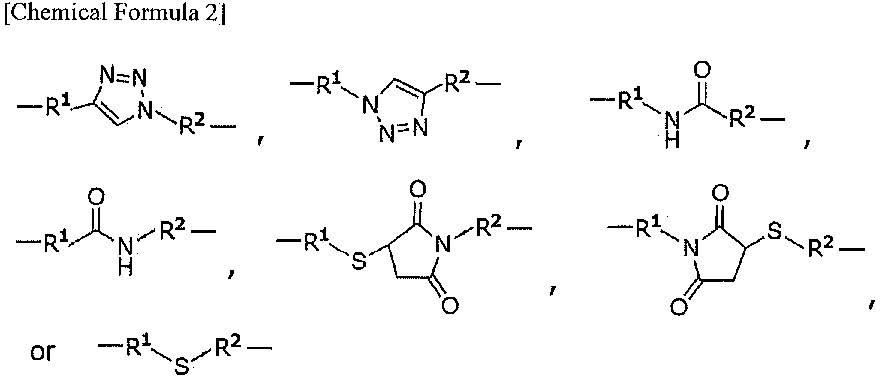 wherein R¹ represents a hydrocarbon group having 1 to 10 carbon atoms, and R² represents a single bond or a hydrocarbon group having 1 to 10 carbon atoms.
[4] The anti-PEG antibody-binding material according to any one of [1] to [3], in which m in the formula (1) represents a number of 10 or more. [0011]
[5] A biosensor including the anti-PEG antibody-binding material according to any one of [1] to [4].
[6] An adsorbent including the anti-PEG antibody-binding material according to any one of [1] to [4].
[7] A detection method for an anti-PEG antibody, including:
   incubating, under conditions that allow binding to an anti-PEG antibody, a cellulose oligomer represented by the above general formula (1) with a specimen suspected of containing the anti-PEG antibody;
   incubating, under conditions that allow binding of a secondary antibody to the anti-PEG antibody, the cellulose oligomer after incubation with the specimen with a sample containing the secondary antibody; and
   detecting the anti-PEG antibody contained in the specimen using the cellulose oligomer after incubation with the sample, with the secondary antibody serving as a probe.

### Advantageous Effects of Invention

According to an embodiment of the invention, an anti-PEG antibody-binding material capable of binding to an anti-PEG antibody is provided. The anti-PEG antibody-binding material can be used, for example, in a biosensor for detecting anti-PEG antibodies or an adsorbent for adsorbing anti-PEG antibodies.

### Brief Description of Drawings

[FIG. 1] A conceptual diagram of a cellulose nanosheet (cellulose oligomer assembly) composed of cellulose oligomers constituting an anti-PEG antibody-binding material according to one embodiment.
[FIG. 2] A reaction scheme for a PEG-introduced cellulose oligomer according to one embodiment.
[FIG. 3] An explanatory diagram showing a detection method for an anti-PEG antibody according to one embodiment.
[FIG. 4] A diagram showing the mass spectra of the cellulose oligomer assemblies obtained in Synthesis Examples 1 to 4.
[FIG. 5] A diagram showing the ATR/FT-IR spectra of the cellulose oligomer assemblies obtained in Synthesis Examples 1 to 4.
[FIG. 6] Graphs of the results of the binding evaluation (absorbance at 490 nm) of the anti-PEG antibody (PEG-B-47) for the cellulose oligomer assemblies in Test Example 1.
[FIG. 7] Graphs of the results of the binding evaluation (absorbance at 490 nm) of the anti-PEG antibody (15-2b) for the cellulose oligomer assemblies in Test Example 2.
[FIG. 8] Graphs of the results of the binding evaluation (absorbance at 490 nm) of the anti-PEG antibody (6.3) for the cellulose oligomer assemblies in Test Example 3.

### Description of Embodiments

An anti-PEG antibody-binding material according to this embodiment includes a cellulose oligomer represented by general formula (1) (hereinafter referred to as a PEG-introduced cellulose oligomer). A PEG-introduced cellulose oligomer is a cellulose oligomer having a structure in which glucose is linked by β-1,4-glycosidic bonds, and has a substituent containing a polyethylene glycol chain (PEG chain) introduced into the anomeric carbon of its reducing end. Specifically, a PEG chain terminated with a methoxy group is bonded via a linking group A to the oxygen atom bonded to the anomeric carbon. As a result of interposing the linking group A in this way, PEG chains of various chain lengths can be introduced into cellulose oligomers.

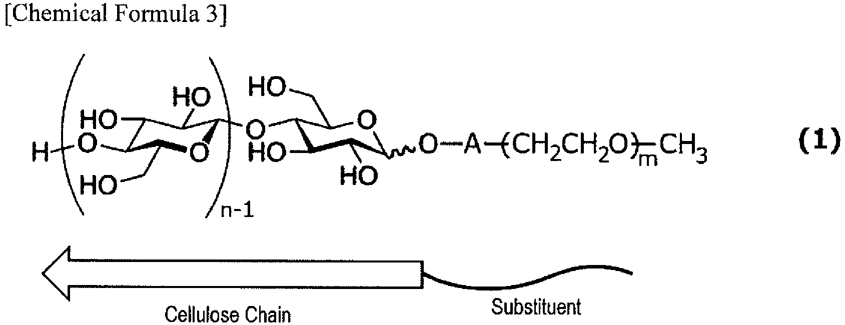

In formula (1), n represents a number of 6 to 16, m represents a number of 3 or more, and A represents a divalent organic group. In addition, the wavy line in formula (1) indicates that the stereochemical configuration at the anomeric position of the reducing end is α-form, β-form, or a mixture thereof.

The above n represents the average degree of polymerization (DP) of the cellulose oligomer, and is 6 or more and 16 or less, n may be 6 to 14, 6.5 to 13, or 7 to 12. The average degree of polymerization of the cellulose oligomer is a weighted average degree of polymerization (number average degree of polymerization) according to the ratio of the number of molecules among cellulose oligomers having different degrees of polymerization. Incidentally, the degree of polymerization of each PEG-introduced cellulose oligomer is not particularly limited, and may be, for example, 4 to 20, 5 to 16, or 6 to 13.

The above m represents the average degree of polymerization (DP) of the PEG chain and is 3 or more. m can be set depending on the selectivity of the anti-PEG antibody to be bound. For example, when m is 10 or more, a backbone-selective anti-PEG antibody is more likely to bind thereto. The upper limit of m is not particularly set, but is preferably 500 or less, for example. In one embodiment, m may be 3 to 500, 10 to 250, 16 to 150, 18 to 100, or 20 to 50. The average degree of polymerization of the PEG chain is a weighted average degree of polymerization according to the ratio of the number of molecules among PEG chains having different degrees of polymerization. Incidentally, in the case where there is no distribution in the molecular weights of PEG chains (i.e., in the case where the polydispersity is 1), m simply represents the degree of polymerization and takes an integer value.

The above A represents a divalent organic group (linking group) that connects the oxygen atom bonded to the anomeric carbon of the reducing end of the cellulose oligomer and the PEG chain (i.e., (CH₂CH₂O)ₘ) and that has 1 to 24 carbon atoms and contains at least one heteroatom selected from the group consisting of a nitrogen atom, an oxygen atom, and a sulfur atom.

The linking group A preferably has 1 to 20 carbon atoms, more preferably 2 to 15 carbon atoms, and still more preferably 2 to 10 carbon atoms. As the group containing a heteroatom contained in the linking group A, for example, a triazole group, an amide group, a thioether group, a maleimide group, an ether group, an ester group, and the like can be mentioned, and via such a heteroatom-containing group, the cellulose oligomer and the PEG chain are linked. Among these, from the viewpoints of the ease of synthesis, the stability of bonding, and the like, it is preferable that the linking group A contains a triazole group, an amide group, or a thioether group.

In one embodiment, A in formula (1) preferably represents a divalent organic group having 2 to 10 carbon atoms and containing at least one member selected from the group consisting of a triazole group, an amide group, and a thioether group.

More specifically, A in formula (1) preferably represents

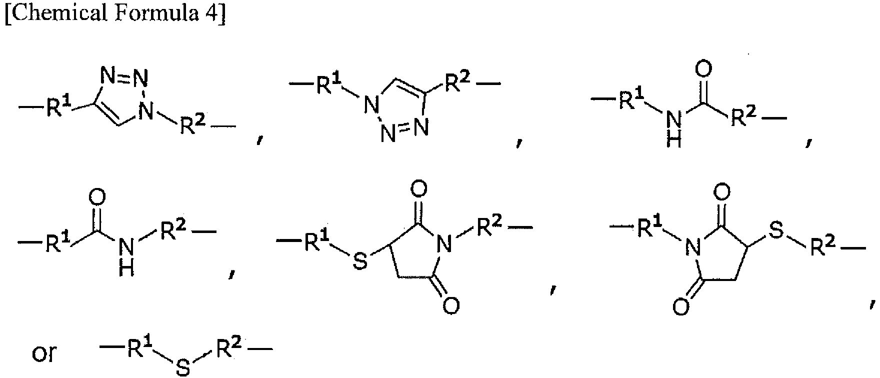

Here, R¹ represents a hydrocarbon group having 1 to 10 carbon atoms (divalent hydrocarbon group), and is bonded to the oxygen atom bonded to the anomeric carbon of the reducing end. R¹ is preferably a linear or branched alkanediyl group, and more preferably a linear alkanediyl group. The hydrocarbon group of R¹ preferably has 1 to 6 carbon atoms, more preferably 1 to 3 carbon atoms.

R² represents a single bond or a hydrocarbon group having 1 to 10 carbon atoms, and is bonded to the PEG chain. R² is preferably a single bond or a linear or branched alkanediyl group, and more preferably a single bond (i.e., a direct bond). The hydrocarbon group of R² preferably has 1 to 6 carbon atoms, more preferably 1 to 3 carbon atoms.

In one embodiment, the PEG-introduced cellulose oligomer is preferably represented by the following general formula (2). In this case, the stereochemical configuration at the anomeric position of the reducing end is β-form. m, n, R¹, and R² in formula (2) are as defined above.

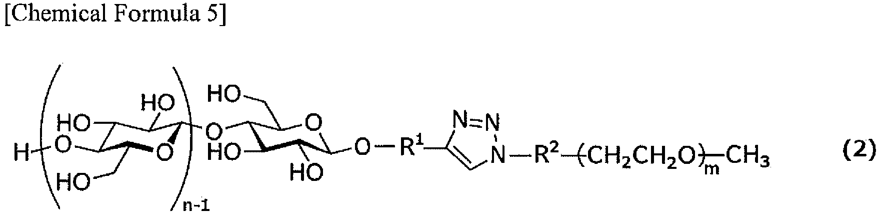

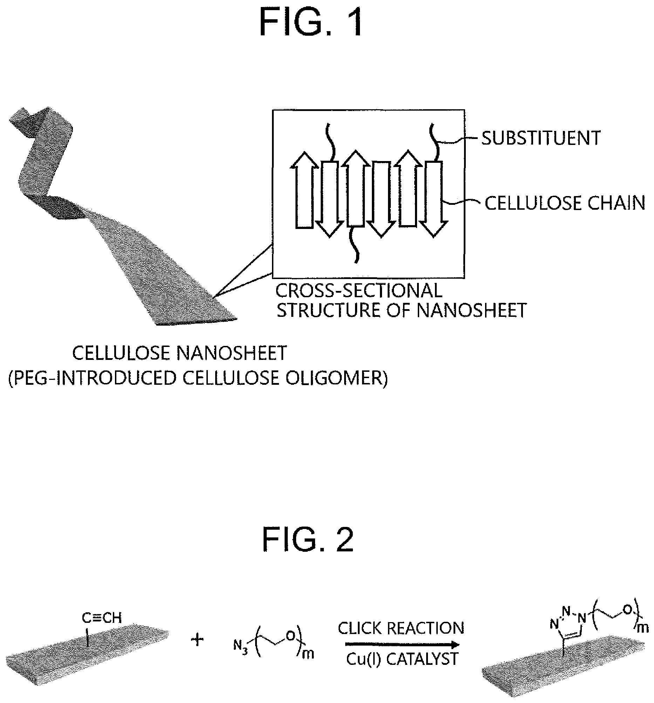

The PEG-introduced cellulose oligomer may be contained in a cellulose oligomer assembly having a cellulose type II crystal structure. The cellulose oligomer assembly may have a sheet-like structure (cellulose nanosheet). Here, the sheet-like structure is a concept that encompasses a ribbon-like structure (cellulose nanoribbon) as shown in FIG. 1.

While naturally occurring cellulose chains have a cellulose type I crystal structure with parallel arrangement, artificially synthesized cellulose oligomers generally form a cellulose type II crystal structure that is thermodynamically stable. At this time, the substituent at the end of a cellulose chain does not affect the crystal form, and substituent-bearing cellulose oligomers are arranged in the thickness direction of a cellulose nanosheet to form lamellar crystals, forming a structure in which the substituents are exposed on the surface of the sheet.

Specifically, as one embodiment, FIG. 1 shows a structure in which PEG chain-containing substituents are introduced into some of the cellulose oligomers that constitute a cellulose nanosheet. In FIG. 1, as appended below the formula (1), for a PEG-introduced cellulose oligomer, the cellulose chain is shown as an arrow pointing from the reducing end to the non-reducing end, and the PEG chain-containing substituent is shown as a wavy line. In addition, for a cellulose oligomer having no PEG chain introduced, the cellulose chain is shown as an arrow pointing from the reducing end to the non-reducing end. As shown by the cross-sectional structure in FIG. 1, cellulose oligomers including PEG-introduced cellulose oligomers are arranged in the film thickness direction such that the directions of the above arrows alternate, forming a structure in which the PEG chain-containing substituents are exposed on the surface of the sheet.

The method for synthesizing a PEG-introduced cellulose oligomer is not particularly limited, and may be as follows. That is, for example, using a cellulose oligomer having a functional group introduced at the anomeric position of the reducing end, a cellulose nanosheet is prepared from such a functional group-introduced cellulose oligomer. A compound having a PEG chain (PEG compound) is allowed to react with the functional group exposed on the surface of the cellulose nanosheet, whereby a PEG chain can be introduced into the cellulose oligomer via the linking group A. As a result of using a cellulose nanosheet that has a functional group exposed on the sheet surface and introducing a PEG chain through a reaction with the functional group in this way, a PEG chain with a long chain length can be introduced. This allows binding to an anti-PEG antibody that specifically binds to a long PEG chain.

As the reaction between a functional group and a compound having a PEG chain, for example, a click reaction between an azide group and an alkynyl group, an amidation reaction between a carboxy group and an amino group, an esterification reaction between a carboxy group and a hydroxy group, a thiol-ene reaction between a thiol group and a carbon-carbon double bond, a reaction between a thiol group and an organic halide, and the like can be mentioned.

FIG. 2 is a reaction scheme for a PEG-introduced cellulose oligomer according to one embodiment. Using a cellulose nanosheet with an ethynyl group exposed on the surface as a functional group, a PEG compound having an azide group is subjected to a click reaction therewith using a metal catalyst such as a copper catalyst. As a result, a PEG chain is introduced into the cellulose oligomer via a linking group A containing a triazole group. This can be expressed by a chemical formula as shown in the following reaction formula (3). In reaction formula (3), a cellulose oligomer having a propargyl group (CEL-C≡CH) and methoxypolyethylene glycol azide (mPEG azide) are subjected to a click reaction.

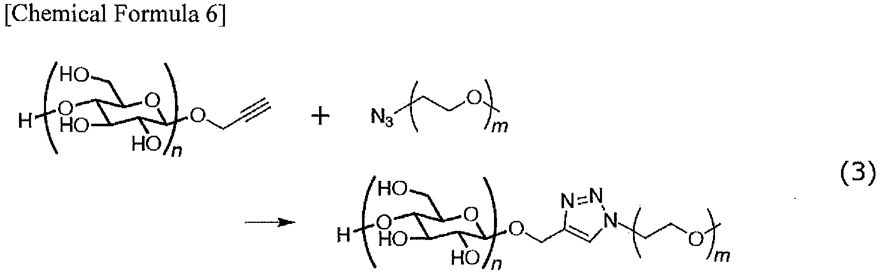

In reaction formula (3), the functional group of the cellulose oligomer is an alkynyl group, and the functional group of the PEG compound is an azide group, but this may be reversed. That is, as shown in the following reaction formula (4), it is also possible that a cellulose oligomer having an azide group as a functional group and a PEG compound having an ethynyl group are subjected to a click reaction.

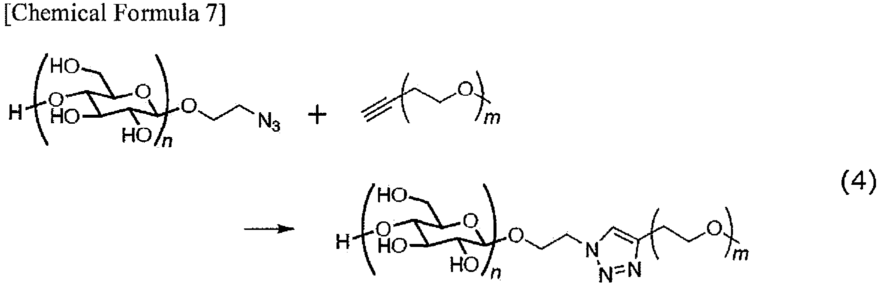

As another embodiment, as shown in the following reaction formula (5), it is also possible that a cellulose oligomer having an amino group as a functional group and a PEG compound having a carboxy group are subjected to an amidation reaction, thereby introducing a PEG chain into the cellulose oligomer via a linking group A containing an amide group.

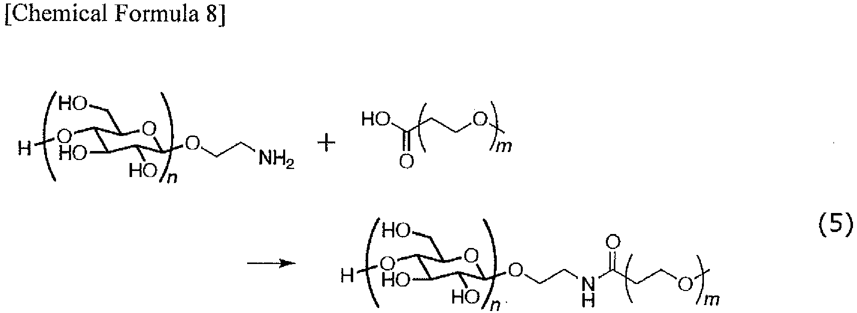

In reaction formula (5), the functional group of the cellulose oligomer is an amino group, and the functional group of the PEG compound is a carboxy group, but this may be reversed. That is, as shown in the following reaction formula (6), it is also possible that a cellulose oligomer having a carboxy group as a functional group and a PEG compound having an amino group are subjected to an amidation reaction.

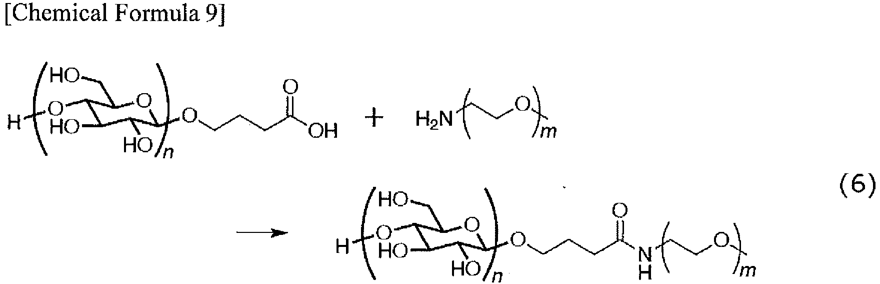

As another embodiment, as shown in the following reaction formula (7), it is also possible that a cellulose oligomer having a thiol group as a functional group and a PEG compound having a maleimide group are subjected to a thiol-ene reaction, thereby introducing a PEG chain into the cellulose oligomer via a linking group A containing a thioether group.

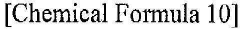

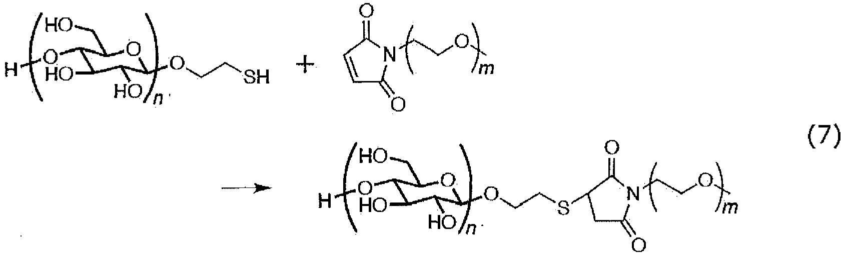

In reaction formula (7), the functional group of the cellulose oligomer is a thiol group, and the functional group of the PEG compound is a maleimide group, but this may be reversed. That is, as shown in the following reaction formula (8), it is also possible that a cellulose oligomer having a maleimide group as a functional group and a PEG compound having a thiol group are subjected to a thiol-ene reaction.

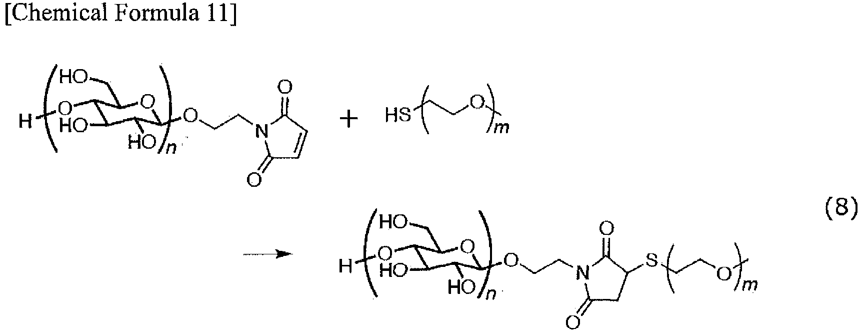

As another embodiment, as shown in the following reaction formula (9), it is also possible that a cellulose oligomer having a thiol group as a functional group is allowed to react with a PEG compound having a halogen atom, thereby introducing a PEG chain into the cellulose oligomer via a linking group A containing a thioether group.

### [Chemical Formula 12]

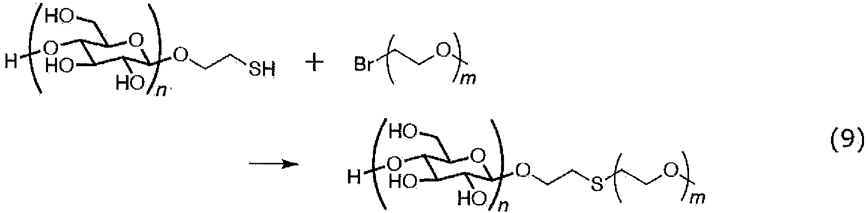

The anti-PEG antibody-binding material according to this embodiment includes a PEG-introduced cellulose oligomer, which is preferably present in the form of a cellulose oligomer assembly such as a cellulose nanosheet. That is, in one embodiment, the anti-PEG antibody-binding material preferably includes a cellulose oligomer assembly containing a PEG-introduced cellulose oligomer. Cellulose oligomers constituting the cellulose oligomer assembly may entirely be PEG-introduced cellulose oligomers, but may also include cellulose oligomers having no PEG chain introduced together with PEG-introduced cellulose oligomers.

In the case where a cellulose oligomer assembly composed of a functional group-introduced cellulose oligomer is used, and a PEG compound is allowed to react therewith as described above, the anti-PEG antibody-binding material may include a cellulose oligomer assembly containing a PEG-introduced cellulose oligomer and a functional group-introduced cellulose oligomer. In the cellulose oligomer assembly, the proportion of PEG-introduced cellulose oligomers in all cellulose oligomers constituting the assembly (PEG introduction rate) is not particularly limited, and may be, for example, 1 mol% or more, preferably 5 to 80 mol%, more preferably 7 to 60 mol%, and still more preferably 10 to 50 mol%.

The PEG-introduced cellulose oligomer described above binds to an anti-PEG antibody such as a backbone-selective anti-PEG antibody or a methoxy-terminated PEG-selective anti-PEG antibody, and does not bind to a secondary antibody used as a probe or other proteins such as BSA (bovine serum albumin) and lysozyme. Therefore, it can be used as an anti-PEG antibody-binding material that can detect or adsorb anti-PEG antibodies.

As anti-PEG antibodies to which the PEG-introduced cellulose oligomer can bind, backbone-selective anti-PEG antibodies and/or methoxy-terminated PEG-selective anti-PEG antibodies can be mentioned. The class of anti-PEG antibodies is not particularly limited, and IgG, IgG1, IgG2b, IgM, and the like can be mentioned.

A backbone-selective anti-PEG antibody is a monoclonal antibody that recognizes the oxyethylene repeating structure, which is the backbone of the PEG structure, and specifically binds to such PEG chains. The molecular weight of PEG chains to which the antibody can specifically bind varies depending on the type of anti-PEG antibody. Therefore, the average degree of polymerization m of the PEG chain in the formula (1) may be set depending on the type of anti-PEG antibody to be detected or adsorbed.

For example, an anti-PEG antibody "6.3" specifically binds to the backbone of a PEG chain of 750 Da (DP = 16) or more; therefore, when this antibody is to be detected or adsorbed, the average degree of polymerization m of the PEG chain in formula (1) may be set to 16 or more. For example, an anti-PEG antibody "AGP4" specifically binds to a PEG chain having 2,000 Da (DP = 45) or more; therefore, when this antibody is to be detected or adsorbed, the average degree of polymerization m of the PEG chain in formula (1) may be set to 45 or more.

A methoxy-terminated PEG-selective anti-PEG antibody is a monoclonal antibody that recognizes a PEG chain terminated with a methoxy group and specifically binds to such PEG chains. A methoxy-terminated PEG-selective anti-PEG antibody may also bind to PEG chains terminated with an ethoxy group or a butoxy group, which is more hydrophobic. The molecular weight of PEG chains to which the antibody can specifically bind varies depending on the type of anti-PEG antibody. Therefore, the average degree of polymerization m of the PEG chain in the formula (1) may be set depending on the type of anti-PEG antibody to be detected or adsorbed.

For example, an anti-PEG antibody "15-2b" specifically binds to a PEG chain having 560 Da (DP = 12) or more and terminated with a methoxy group; therefore, when this antibody is to be detected or adsorbed, the average degree of polymerization m of the PEG chain in formula (1) may be set to 12 or more. For example, an anti-PEG antibody "PEG-B-47" specifically binds to a PEG chain having a DP of 3 or more and terminated with a methoxy group; therefore, when this antibody is to be detected or adsorbed, the average degree of polymerization m of the PEG chain in formula (1) may be set to 3 or more.

The anti-PEG antibody-binding material according to the embodiment includes a PEG-introduced cellulose oligomer, and is thus capable of recognizing and binding to an anti-PEG antibody. Therefore, it can be used, for example, in a biosensor for detecting anti-PEG antibodies, an adsorbent for adsorbing anti-PEG antibodies, and the like.

The anti-PEG antibody-binding material may be composed only of a PEG-introduced cellulose oligomer or a cellulose oligomer assembly containing the same, or may also contain, without impairing its effect, other components such as proteins, peptides, nucleic acids, lipids, sugars, cells, amino acids, metal ions, and organic solvents.

The PEG-introduced cellulose oligomer or the cellulose oligomer assembly is a substance that is insoluble in water, and when prepared by the above synthesis method, it is obtained as an aqueous dispersion of a cellulose nanosheet containing a PEG-introduced cellulose oligomer. The anti-PEG antibody-binding material according to one embodiment may be a dry powder obtained by removing water from the aqueous dispersion, and it is also possible that the dry powder is used as it is or mixed with other components such as proteins in powder form, thereby preparing an anti-PEG antibody-binding material in powder form. When used, the anti-PEG antibody-binding material in powder form can be easily dispersed in water by adding water, and an aqueous dispersion can thus be prepared.

An anti-PEG antibody-binding material according to one embodiment may be an aqueous dispersion in which the PEG-introduced cellulose oligomer or the cellulose oligomer assembly is dispersed in water. In that case, a buffer solution prepared by adding a buffering agent to water may be used as a dispersion medium. That is, the aqueous dispersion may be a dispersion of the PEG-introduced cellulose oligomer or the cellulose oligomer assembly in a buffer solution. The content (concentration) of the PEG-introduced cellulose oligomer in the aqueous dispersion is not particularly limited, and may be, for example, 0.01 to 5% (w/v), or 0.03 to 0.3% (w/v).

As used herein, "% (w/v)" is a mass/volume percent concentration, which is the mass (g) of the target substance in a volume of 100 mL.

The buffering agent is not particularly limited, and, for example, buffering agents constituting phosphate buffered saline (PBS), 2-morpholinoethanesulfonic acid (MES) buffer solution, trishydroxymethylaminomethane (Tris) buffer solution, 3-morpholinopropanesulfonic acid (MOPS) buffer solution, 4-(2-hydroxyethyl)-1-piperazineethanesulfonic acid (HEPES) buffer solution, and the like can be mentioned.

An anti-PEG antibody-binding material according to one embodiment may be obtained by immobilizing the PEG-introduced cellulose oligomer or the cellulose oligomer assembly on the surface of a substrate such as plastic or glass.

In one embodiment, a biosensor containing the anti-PEG antibody-binding material is used to perform a bioassay to detect and quantify anti-PEG antibodies. The biosensor refers to each element used to perform the bioassay or a collection or combination of such elements.

Therefore, the biosensor may be composed only of a powder of the PEG-introduced cellulose oligomer or the cellulose oligomer assembly, or may be composed only of an aqueous dispersion of the PEG-introduced cellulose oligomer or the cellulose oligomer assembly, for example. Alternatively, the biosensor may also be a kit (i.e., an anti-PEG antibody detection kit) that combines such a powder or aqueous dispersion of the PEG-introduced cellulose oligomer or the cellulose oligomer assembly with a secondary antibody as a probe, and, further, in the case where the secondary antibody is an enzyme-labeled antibody, it may also be an anti-PEG antibody detection kit that combines a substrate corresponding to the enzyme.

The biosensor may contain albumin such as BSA, and it is preferable that albumin is added during the incubation of the PEG-introduced cellulose oligomer or the cellulose oligomer assembly with a specimen. In that case, albumin may be contained in the anti-PEG antibody-binding material, or may also be contained in the anti-PEG antibody detection kit separately from the anti-PEG antibody-binding material. In the case where albumin is contained in the anti-PEG antibody-binding material, the albumin may be mixed with the PEG-introduced cellulose oligomer or the cellulose oligomer assembly in powder form, or it is also possible that the albumin is dissolved in an aqueous dispersion. The ratio of the PEG-introduced cellulose oligomer to albumin is not particularly limited, and, for example, the PEG-introduced cellulose oligomer/albumin mass ratio may be 0.002/1 to 0.3/1, or 0.01/1 to 0.1/1. Incidentally, in the case of a specimen containing albumin such as blood, additional albumin may or may not be added.

A detection method for an anti-PEG antibody according to one embodiment includes the following steps:
(1) a step of incubating a PEG-introduced cellulose oligomer with a specimen under conditions that allow binding to an anti-PEG antibody;
(2) a step of incubating the PEG-introduced cellulose oligomer after incubation with the specimen with a sample containing a secondary antibody under conditions that allow binding of the secondary antibody; and
(3) a step of detecting an anti-PEG antibody contained in the specimen using the PEG-introduced cellulose oligomer after incubation with the sample, with the secondary antibody serving as a probe.

In step (1), a specimen is added to a container together with an aqueous dispersion of a PEG-introduced cellulose oligomer as the anti-PEG antibody-binding material, mixed to cause dispersion, and then incubated. As the aqueous dispersion, an aqueous dispersion of a cellulose oligomer assembly, which is an assembly containing a PEG-introduced cellulose oligomer, is preferably used.

The specimen is a substance to be tested that is suspected of containing an anti-PEG antibody. For example, in the case of detecting an anti-PEG antibody present in the blood in order to evaluate the impact on the induction of anti-PEG antibodies and the therapeutic efficacy of PEG-modified pharmaceuticals, the specimen may be blood or a dilution thereof.

In step (1), as described above, albumin such as BSA may be added to improve the sensitivity of anti-PEG antibody detection. In that case, the albumin concentration in the system (the albumin concentration excluding albumin originally contained in the specimen) is not particularly limited, but is, for example, preferably 0.05 to 10 mass%, and more preferably 0.5 to 5 mass%.

The incubation conditions in step (1) are not particularly limited as long as the PEG-introduced cellulose oligomer and the anti-PEG antibody can bind to each other, and may be, for example, 4 to 60°C and 1 minute to 2 hours. The concentration of the PEG-introduced cellulose oligomer in the system is not particularly limited, and may be, for example, 0.001 to 1% (w/v), or 0.01 to 0.1% (w/v).

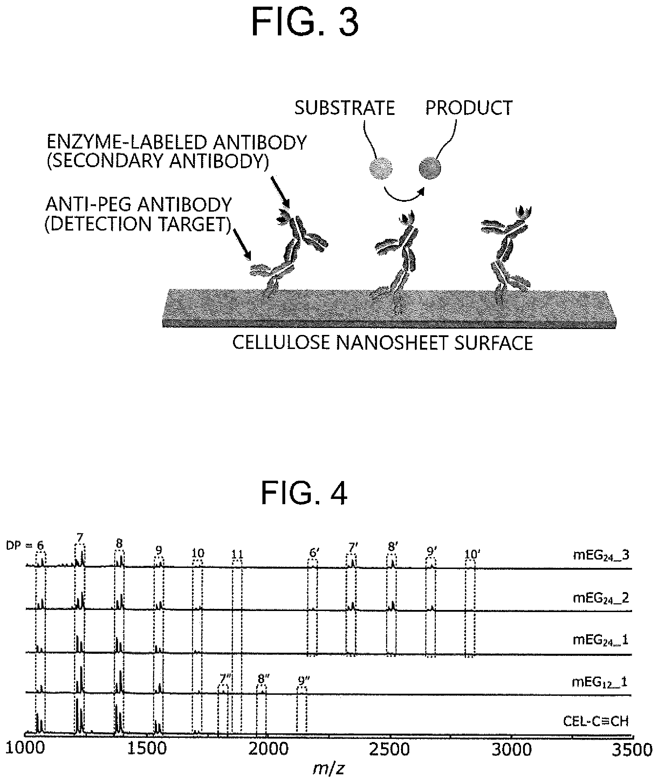

In step (1), the anti-PEG antibody, which is the detection target, is bound to the PEG-introduced cellulose oligomer in this way (see FIG. 3). Subsequently, the PEG-introduced cellulose oligomer having the anti-PEG antibody bound thereto is separated from the specimen by centrifugation and washing.

In step (2), a sample containing a secondary antibody is added to the PEG-introduced cellulose oligomer having the anti-PEG antibody bound thereto (preferably a cellulose oligomer assembly containing the same), mixed to cause dispersion, and then incubated.

The sample containing a secondary antibody may be an aqueous solution of the secondary antibody. The secondary antibody is not particularly limited as long as it specifically binds to the anti-PEG antibody and thus serves as a probe, and may be, for example, an antibody labeled with an enzyme, a radioisotope, a fluorescent dye, or the like.

The incubation conditions in step (2) are not particularly limited as long as the anti-PEG antibody and the secondary antibody can bind to each other, and may be, for example, 4 to 60°C and 1 minute to 2 hours. The concentration of the secondary antibody in the system is not particularly limited, and may be, for example, 100 to 1,000 ng/mL.

In step (2), the secondary antibody as a probe is bound to the anti-PEG antibody that has been bound to the PEG-introduced cellulose oligomer in this way (see FIG. 3). Subsequently, the PEG-introduced cellulose oligomer having the secondary antibody bound thereto (preferably a cellulose oligomer assembly containing the same) is separated from the sample containing the secondary antibody by centrifugation and washing.

In step (3), the anti-PEG antibody contained in the specimen is detected and quantified using the PEG-introduced cellulose oligomer having the secondary antibody bound thereto, with the secondary antibody serving as a probe.

For example, in the case where an enzyme-labeled antibody is used as the secondary antibody, as shown in FIG. 3, a substrate corresponding to the enzyme is added to the cellulose oligomer assembly having the secondary antibody bound thereto, and an enzymatic reaction is performed. The coloring matter of the resulting product is detected and quantified by absorbance (color development), fluorescence, chemiluminescence, or the like.

In another embodiment, an adsorbent containing the anti-PEG antibody-binding material can be used to adsorb an anti-PEG antibody, thereby removing or purifying the anti-PEG antibody.

The adsorbent may be composed of a powder or aqueous dispersion of a PEG-introduced cellulose oligomer or a cellulose oligomer assembly containing the same, and other optional components may also be contained therein.

In the case where the adsorbent is used to remove an anti-PEG antibody, for example, the adsorbent is added to a liquid containing an anti-PEG antibody to bring the anti-PEG antibody into contact with the PEG-introduced cellulose oligomer, allowing the two to bind to each other. Subsequently, the adsorbent to which the anti-PEG antibody has been adsorbed is removed from the liquid by centrifugation or the like, whereby the anti-PEG antibody can be removed from the liquid.

In the case where the adsorbent is used to purify an anti-PEG antibody, for example, the adsorbent is added to a liquid containing impurities along with an anti-PEG antibody to bring the anti-PEG antibody into contact with the PEG-introduced cellulose oligomer, allowing the two to bind to each other. Subsequently, the adsorbent to which the anti-PEG antibody has been adsorbed is separated and recovered from the liquid by centrifugation or the like. The recovered adsorbent is subjected, for example, to an acid treatment to dissociate the binding between the anti-PEG antibody and the PEG-introduced cellulose oligomer. As a result, the anti-PEG antibody can be separated and recovered from the adsorbent, and a high-purity anti-PEG antibody can be obtained.

### Examples

Hereinafter, the invention will be described in detail with reference to examples, but is not limited thereto.

### [Enzymatic Synthesis of Cellulose Oligomer Having Propargyl Group]

Predetermined amounts of a 1M aqueous solution of α-glucose-1-phosphate (αG1P), a 1M 2-[4-(2-hydroxyethyl)-1-piperazinyl]ethanesulfonic acid (HEPES) buffer solution (pH 7.5), a 100 µM EDTA solution, and a 1M aqueous solution of 2-propynyl-β-D-glucoside (Glc-C≡CH) were mixed, and, using ultrapure water, prepared such that αG1P was 200 mM, HEPES was 500 mM, EDTA was 50 µM, and Glc-C≡CH was 50 mM. Here, "M" represents "mol/L". Cellodextrin phosphorylase (CDP) was added to the obtained solution to a concentration of 1 U/mL, followed by incubation at 60°C for 3 days. A liquid containing the precipitated product was centrifuged (20,400 × g, 5 minutes, 4°C), the supernatant was removed, and then ultrapure water was added to redisperse the product, followed by centrifugation (under the same conditions); this procedure was repeated five times to obtain a cellulose oligomer having a propargyl group (CEL-C≡CH).

Here, the above 2-propynyl-β-D-glucoside was synthesized as follows. Tetra-O-acetyl-2-propynyl-β-D-glucoside was synthesized according to a previous report (H.B. Mereyala et al., Carbohydr. Polym., 1998, 307, 351-354). Next, 0.97 g of tetra-O-acetyl-propargyl-β-D-glucoside and 0.034 g of sodium methoxide were allowed to react in the presence of 25 mL of methanol and 25 mL of tetrahydrofuran at 25°C for 12 hours or more, followed by purification by silica gel column chromatography, thereby giving 2-propynyl-β-D-glucoside.

The fact that the product of the enzymatic synthesis was a cellulose oligomer having a propargyl group at one end was confirmed with a proton nuclear magnetic resonance (NMR) spectrometer (AVANCE III HD500 (Bruker Biospin, magnetic field strength: 500 MHz, 16 scans)) using a sample prepared by dissolving the product in a 4% sodium deuteroxide solution in heavy water at a concentration of 2 mass% or more.

The mass spectrum of the product was obtained by the matrix-assisted laser desorption/ionization time-of-flight mass spectrometry (MALDI-TOF-MS) method, and the cellulose oligomer was found to have an average degree of polymerization of 7.5 and a polydispersity index (PDI) of 1.02. The measurement conditions for MALDI-TOF-MS and the calculation methods for the average degree of polymerization and the polydispersity index PDI are as follows.

The measurement was performed using a MALDI-TOF MS apparatus (AXIMA-performance (manufactured by Shimadzu Corporation)) under the following conditions: acceleration voltage 20 kV, Linear (positive) ion mode, Mass Range 1 - 4000, Max Leaser Rap Rate 10, Power 100, profiles 100, shots 2, Ion Gate Blank 500 Da, and Pulsed Extraction optimized at 1,500 Da. Based on the integral values of the peaks of the sodium or potassium adducts of the cellulose oligomer (CEL-C=CH) having a degree of polymerization of 6 to 11 and having a propargyl group at one end, the average degree of polymerization and the polydispersity index (PDI) were calculated.

### [Introduction of PEG Chain into Cellulose Oligomer]

According to the reaction scheme shown in FIG. 2 and the reaction shown in the reaction formula (3), the cellulose oligomer having a propargyl group (CEL-C=CH) was subjected to a click reaction with methoxypolyethylene glycol azide (mPEG azide). In the following Synthesis Examples 1 to 4, four types of PEG-introduced cellulose oligomers with different PEG incorporation rates were prepared.

### [Synthesis Example 1]

A CEL-C=CH aqueous dispersion, 40 mM methyl-PEG24-azide (mPEG azide of Reaction Formula (3) wherein m = 24), DMSO (SP grade), a 20 mM aqueous CuSO₄ solution, an 8 mM aqueous sodium ascorbate solution, and ultrapure water were added to a 5-mL tube such that the CEL-C=CH was 2 mg/mL, methyl-PEG24-azide was 2 mM, CuSO₄ was 0.5 mM, and ascorbic acid was 0.2 mM, and then stirred at 25°C for 1 hour to effect the reaction. At this time, water/DMSO (volume ratio) was 1/1, and the volume of the reaction solution was 5,000 µL. The liquid after the reaction was centrifuged (20,400 × g, 5 minutes, 4°C), the supernatant was removed, and then ultrapure water was added to redisperse the product, followed by centrifugation (under the same conditions); this procedure was repeated five times to obtain a cellulose oligomer assembly (mEG₂₄_1) of Synthesis Example 1.

### [Synthesis Example 2]

A cellulose oligomer assembly (mEG_{24_}2) of Synthesis Example 2 was obtained in the same manner as in Synthesis Example 1, except that the amounts added were set such that methyl-PEG24-azide was 4 mM, CuSO₄ was 1.0 mM, and ascorbic acid was 0.4 mM.

### [Synthesis Example 3]

A cellulose oligomer assembly (mEG₂₄_3) of Synthesis Example 3 was obtained in the same manner as in Synthesis Example 1, except that the amounts added were set such that methyl-PEG24-azide was 6 mM, CuSO₄ was 1.5 mM, and ascorbic acid was 0.6 mM.

### [Synthesis Example 4]

A CEL-C=CH aqueous dispersion, 40 mM methyl-PEG12-azide (mPEG azide of Reaction Formula (3) wherein m = 12), DMSO (SP grade), a 20 mM aqueous CuSO₄ solution, an 8 mM aqueous sodium ascorbate solution, and ultrapure water were added to a 5-mL tube such that the CEL-C=CH was 2 mg/mL, methyl-PEG12-azide was 2 mM, CuSO₄ was 0.5 mM, and ascorbic acid was 0.2 mM, and then stirred at 25°C for 1 hour to effect the reaction. At this time, water/DMSO (volume ratio) was 1/1, and the volume of the reaction solution was 2,500 µL. The liquid after the reaction was centrifuged (20,400 × g, 5 minutes, 4°C), the supernatant was removed, and then ultrapure water was added to redisperse the product, followed by centrifugation (under the same conditions); this procedure was repeated five times to obtain a cellulose oligomer assembly (mEG₁₂_1) of Synthesis Example 4.

The mass spectra of the cellulose oligomer assemblies (mEG_{24_}1 to 3 and mEG₁₂_1) were obtained by the MALDI-TOF-MS method. The results are as shown in FIG. 4. In FIG. 4, the peaks enclosed by dashed lines at DP = 6 to 11 are peaks of the CEL-C=CH having no PEG chain introduced, and the numerical values 6 to 11 each represent the degree of polymerization of cellulose in the CEL-C=CH. The peaks enclosed by dashed lines at DP = 6' to 10' are peaks of the PEG-introduced cellulose oligomers having a PEG chain with m = 24 introduced, and the numerical values 6' to 10' each represent the degree of polymerization of cellulose in the PEG-introduced cellulose oligomers. The peaks enclosed by dashed lines at DP = 7" to 9" are peaks of the PEG-introduced cellulose oligomer having a PEG chain with m = 12 introduced, and the numerical values 7" to 9" each represent the degree of polymerization of cellulose in the PEG-introduced cellulose oligomer.

From the integral values of the peaks of the PEG-introduced cellulose oligomers and the peaks of the CEL-C=CH, the PEG introduction rate of each cellulose oligomer assembly (mEG₂₄_1 to 3 and mEG₁₂_1) was determined. The results are shown in Table 1 below. Incidentally, the PEG introduction rate is the proportion (mol%) of PEG-introduced cellulose oligomers in all cellulose oligomers constituting the cellulose oligomer assembly.

**[Table 1]**

| | PEG Introduction Rate |
|---|---|
| mEG₂₄_1 | 10% |
| mEG₂₄_2 | 30% |
| mEG₂₄_3 | 44% |
| mEG₁₂_1 | 10% |

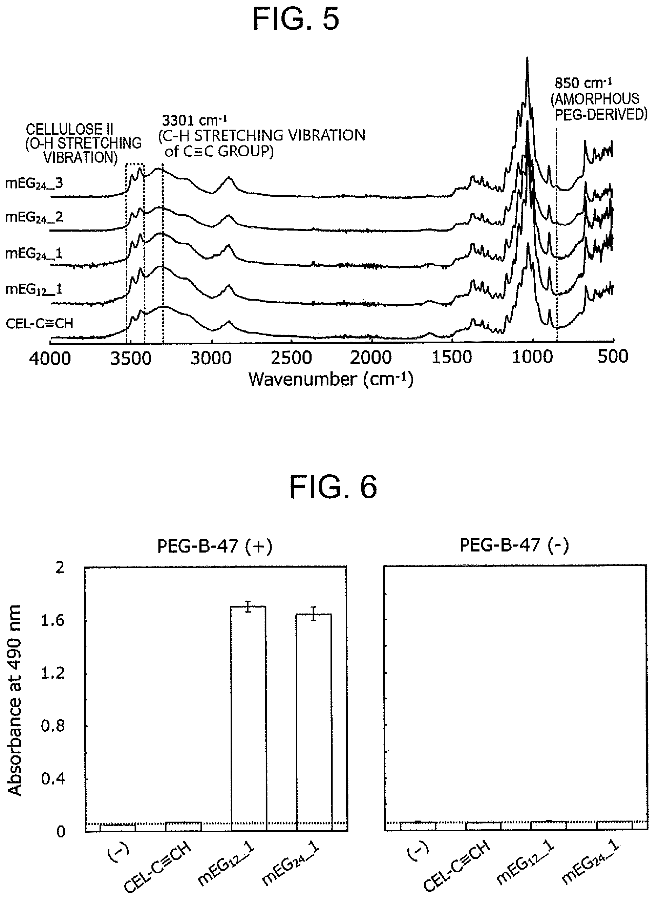

The cellulose oligomer assemblies (mEG₂₄_1 to 3 and mEG₁₂_1) were subjected to ATR/FT-IR measurement, and the spectra shown in FIG. 5 were obtained. The obtained spectra showed peaks derived from hydroxyl groups of cellulose forming the cellulose type II crystal structure, peaks derived from acetylene groups, and peaks derived from amorphous PEG chains. This indicates that a click reaction does not affect the crystal structure of cellulose, and that the PEG chains exposed on the surface of the cellulose nanosheets are in an amorphous state.

### [Reagent]

The reagents BSA and OPD used in the following test examples are as follows.
- BSA: Bovine serum albumin, manufactured by FUJIFILM Wako Pure Chemical Corporation, protease-free
- OPD: o-Phenylenediamine dihydrochloride, manufactured by Nacalai Tesque, for water quality analysis

### [Test Example 1: Binding Evaluation of Anti-PEG Antibody (PEG-B-47)]

As an anti-PEG antibody to serve as the primary antibody, PEG-B-47 (manufactured by abcam) was used. PEG-B-47 is a methoxy-terminated PEG-selective monoclonal anti-PEG antibody (animal species: rabbit, class: IgG), which specifically binds to a PEG chain having 140 Da (DP = 3) or more and terminated with a methoxy group. PEG-B-47 was diluted with a 3.3 mass% BSA/PBS (pH 7.4) solution to prepare a primary antibody solution with a PEG-B-47 content of 110 ng/mL.

The cellulose oligomer assemblies obtained in Synthesis Examples 1 and 4 (mEG₂₄_1 and mEG₁₂_1) were each dispersed in PBS at 5 mg/mL to prepare two types of cellulose dispersions according to examples. In addition, the cellulose oligomer assembly having a propargyl group (CEL-C=CH) synthesized above was dispersed in PBS at 5 mg/mL to prepare a cellulose dispersion liquid according to a comparative example.

As the secondary antibody used to evaluate the binding of the anti-PEG antibody, an HRP-labeled anti-rabbit IgG antibody (manufactured by GeneTex) was used. The antibody was diluted with a 3.3 mass% BSA/PBS solution to prepare a secondary antibody solution with a secondary antibody content of 440 ng/mL.

### 1. Antigen-Antibody Reaction of Primary Antibody

10 µL of each cellulose dispersion was added to a 0.2-mL PCR tube, 100 µL of the primary antibody solution was further added, and the mixture was dispersed, followed by incubation at 25°C for 1 hour to perform an antigen-antibody reaction. This antigen-antibody reaction system of the primary antibody had a cellulose oligomer assembly concentration of 0.45 mg/mL, a primary antibody concentration of 100 ng/mL, and a BSA concentration of 3 mass%. After incubation, centrifugation was performed (15,000 rpm, 25°C, 5 minutes), and the supernatant was removed, followed by redispersion in 100 µL of PBS; this procedure was repeated three times for washing.

### 2. Antigen-Antibody Reaction of Secondary Antibody

To the PCR tube after washing, that is, after centrifugation and the removal of the supernatant, 100 µL of the secondary antibody solution was added, and the mixture was dispersed, followed by incubation at 25°C for 1 hour to perform an antigen-antibody reaction. This antigen-antibody reaction system of the secondary antibody had a cellulose oligomer assembly concentration of 0.45 mg/mL and a secondary antibody concentration of 400 ng/mL. After incubation, centrifugation was performed (15,000 rpm, 25°C, 5 minutes), and the supernatant was removed, followed by redispersion in 100 µL of PBS; this procedure was repeated three times for washing.

### 3. Enzymatic Reaction

Next, to the PCR tube after washing, that is, after centrifugation and the removal of the supernatant, 100 µL of a substrate solution was added, and the mixture was dispersed, followed by incubation at 25°C for 30 minutes. As the substrate solution, a citrate-phosphate buffer (pH 4.0) containing 2 mg/mL of OPD and 0.11 v/v% of hydrogen peroxide was used. After incubation, 100 µL of 3N sulfuric acid was added, followed by centrifugation (15,000 rpm, 25°C, 10 minutes). 100 µL of the supernatant was added to a 96-well plate, and the absorbance at 490 nm was measured using a plate reader ("Synergy H1" manufactured by BioTek).

The results are shown in FIG. 6. In FIG. 6, "mEG₂₄_1" and "mEG₁₂_1" in the graph "PEG-B-47 (+)" on the left represent the results of the above test. The test was performed in three sets of three experiments, and the absorbance was determined as the grand mean of the mean values of the respective sets.

Incidentally, as a control, the reactions 1 to 3 were performed in the same manner as above, except that in the antigen-antibody reaction of the primary antibody described above, 100 µL of a 3.3 mass% BSA/PBS (pH 7.4) solution containing no PEG-B-47 was added instead of 100 µL of the primary antibody solution. The results are shown in the graph "PEG-B-47 (-)" on the right in FIG. 6.

In addition, the reactions 1 to 3 were performed in the same manner as above, except that in the antigen-antibody reaction of the primary antibody described above, 10 µL of PBS containing no cellulose oligomer assembly was added instead of 10 µL of the cellulose dispersion. The results are shown as "(-)" in the graphs "PEG-B-47 (+)" and "PEG-B-47 (-)" in FIG. 6.

As shown in FIG. 6, with the cellulose oligomer assembly CEL-C=CH according to a comparative example, the absorbance was equivalent to that of "(-)" with no cellulose oligomer, and the anti-PEG antibody PEG-B-47 did not bind. In contrast, with the cellulose oligomer assemblies according to examples (mEG₂₄_1 and mEG₁₂_1), the absorbance was significantly higher than that of "(-)", indicating the binding of the anti-PEG antibody PEG-B-47. Therefore, with the cellulose oligomer assemblies according to examples, methoxy-terminated PEG-selective anti-PEG antibodies can be detected.

### [Test Example 2: Binding Evaluation of Anti-PEG Antibody (15-2b)]

As an anti-PEG antibody to serve as the primary antibody, 15-2b (manufactured by Merck) was used. 15-2b is a methoxy-terminated PEG-selective monoclonal anti-PEG antibody (animal species: mouse, class: IgG2b), which specifically binds to a PEG chain having 560 Da (DP = 12) or more and terminated with a methoxy group. 15-2b was diluted with a 3.3 mass% BSA/PBS (pH 7.4) solution to prepare a primary antibody solution with a 15-2b content of 110 ng/mL.

The cellulose oligomer assemblies obtained in Synthesis Examples 1 to 4 (mEG₂₄_1 to 3 and mEG₁₂_1) were each dispersed in PBS at 5 mg/mL to prepare four types of cellulose dispersions according to examples. In addition, the cellulose oligomer assembly having a propargyl group (CEL-C=CH) synthesized above was dispersed in PBS at 5 mg/mL to prepare a cellulose dispersion liquid according to a comparative example.

As the secondary antibody used to evaluate the binding of the anti-PEG antibody, an HRP-labeled anti-mouse IgG antibody (manufactured by Sigma-Aldrich) was used. The antibody was diluted with a 3.3 mass% BSA/PBS solution to prepare a secondary antibody solution with a secondary antibody content of 440 ng/mL.

### 1. Antigen-Antibody Reaction of Primary Antibody

10 µL of each cellulose dispersion was added to a 0.2-mL PCR tube, 100 µL of the primary antibody solution was further added, and the mixture was dispersed, followed by incubation at 25°C for 1 hour to perform an antigen-antibody reaction. This antigen-antibody reaction system of the primary antibody had a cellulose oligomer assembly concentration of 0.45 mg/mL, a primary antibody concentration of 100 ng/mL, and a BSA concentration of 3 mass%. After incubation, centrifugation was performed (15,000 rpm, 25°C, 5 minutes), and the supernatant was removed, followed by redispersion in 100 µL of PBS; this procedure was repeated three times for washing.

### 2. Antigen-Antibody Reaction of Secondary Antibody

To the PCR tube after washing, that is, after centrifugation and the removal of the supernatant, 100 µL of the secondary antibody solution was added, and the mixture was dispersed, followed by incubation at 25°C for 1 hour to perform an antigen-antibody reaction. This antigen-antibody reaction system of the secondary antibody had a cellulose oligomer assembly concentration of 0.45 mg/mL and a secondary antibody concentration of 400 ng/mL. After incubation, centrifugation was performed (15,000 rpm, 25°C, 5 minutes), and the supernatant was removed, followed by redispersion in 100 µL of PBS; this procedure was repeated three times for washing.

### 3. Enzymatic Reaction

Next, to the PCR tube after washing, that is, after centrifugation and the removal of the supernatant, 100 µL of a substrate solution was added, and the mixture was dispersed, followed by incubation at 25°C for 30 minutes. As the substrate solution, a citrate-phosphate buffer (pH 4.0) containing 2 mg/mL of OPD and 0.11 v/v% of hydrogen peroxide was used. After incubation, 100 µL of 3N sulfuric acid was added, followed by centrifugation (15,000 rpm, 25°C, 10 minutes). 100 µL of the supernatant was added to a 96-well plate, and the absorbance at 490 nm was measured using a plate reader ("Synergy H1" manufactured by BioTek).

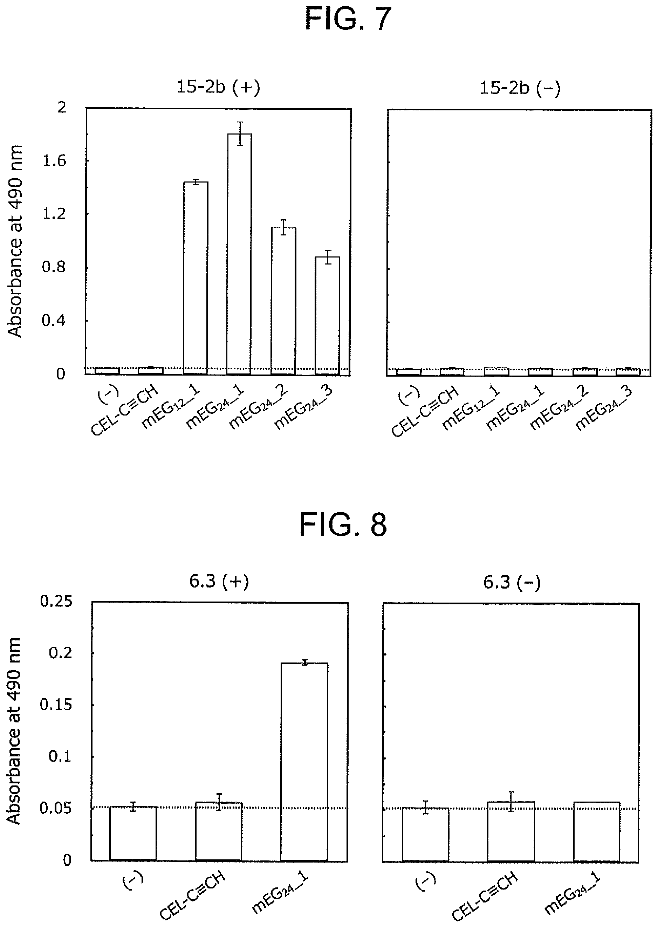

The results are shown in FIG. 7. In FIG. 7, "mEG₂₄_1", "mEG₂₄_2", "mEG₂₄_3", "mEG_{12_}1 ", and "CEL-C≡CH" in the graph "15-2b (+)" on the left represent the results of the above test. The test was performed in three sets of three experiments, and the absorbance was determined as the grand mean of the mean values of the respective sets.

Incidentally, as a control, the reactions 1 to 3 were performed in the same manner as above, except that in the antigen-antibody reaction of the primary antibody described above, 100 µL of a 3.3 mass% BSA/PBS (pH 7.4) solution containing no 15-2b was added instead of 100 µL of the primary antibody solution. The results are shown in the graph "15-2b (-)" on the right in FIG. 7.

In addition, the reactions 1 to 3 were performed in the same manner as above, except that in the antigen-antibody reaction of the primary antibody described above, 10 µL of PBS containing no cellulose oligomer assembly was added instead of 10 µL of the cellulose dispersion. The results are shown as "(-)" in the graphs "15-2b (+)" and "15-2b (-)" in FIG. 7.

As shown in FIG. 7, with the cellulose oligomer assembly CEL-C=CH according to a comparative example, the absorbance was equivalent to that of "(-)" with no cellulose oligomer, and the anti-PEG antibody 15-2b did not bind. In contrast, with the cellulose oligomer assemblies according to examples (mEG₂₄_1 to 3 and mEG₁₂_1), the absorbance was significantly higher than that of "(-)", indicating the binding of the anti-PEG antibody 15-2b. Therefore, with the cellulose oligomer assemblies according to examples, methoxy-terminated PEG-selective anti-PEG antibodies can be detected.

### [Test Example 3: Binding Evaluation of Anti-PEG Antibody (6.3)]

As an anti-PEG antibody to serve as the primary antibody, 6.3 (manufactured by Merck) was used. 6.3 is a backbone-selective anti-PEG antibody (animal species: mouse, class: IgGl), which specifically binds to a PEG chain having 750 Da (DP = 16) or more (Samuel K. Lai, Commun. Chem., 2020, 3, 124). 6.3 was diluted with a 3.3 mass% BSA/PBS (pH 7.4) solution to prepare a primary antibody solution with a 6.3 content of 110 ng/mL. A cellulose dispersion was prepared in the same manner as in Test Example 1 using the cellulose oligomer of Synthesis Example 1. As a secondary antibody solution, the same solution as in Test Example 1 was used.

### 1. Antigen-Antibody Reaction of Primary Antibody

10 µL of each cellulose dispersion was added to a 0.2-mL PCR tube, 100 µL of the primary antibody solution was further added, and the mixture was dispersed, followed by incubation at 25°C for 1 hour to perform an antigen-antibody reaction. This antigen-antibody reaction system of the primary antibody had a cellulose oligomer assembly concentration of 0.45 mg/mL, a primary antibody concentration of 100 ng/mL, and a BSA concentration of 3 mass%. After incubation, centrifugation was performed (15,000 rpm, 25°C, 5 minutes), and the supernatant was removed, followed by redispersion in 100 µL of PBS; this procedure was repeated three times for washing.

### 2. Antigen-Antibody Reaction of Secondary Antibody

To the PCR tube after washing, that is, after centrifugation and the removal of the supernatant, 100 µL of the secondary antibody solution was added, and the mixture was dispersed, followed by incubation at 25°C for 1 hour to perform an antigen-antibody reaction. This antigen-antibody reaction system of the secondary antibody had a cellulose oligomer assembly concentration of 0.45 mg/mL and a secondary antibody concentration of 400 ng/mL. After incubation, centrifugation was performed (15,000 rpm, 25°C, 5 minutes), and the supernatant was removed, followed by redispersion in 100 µL of PBS; this procedure was repeated three times for washing.

### 3. Enzymatic Reaction

Next, to the PCR tube after washing, that is, after centrifugation and the removal of the supernatant, 100 µL of a substrate solution was added, and the mixture was dispersed, followed by incubation at 25°C for 30 minutes. As the substrate solution, a citrate-phosphate buffer (pH 4.0) containing 2 mg/mL of OPD and 0.11 v/v% of hydrogen peroxide was used. After incubation, 100 µL of 3N sulfuric acid was added, followed by centrifugation (15,000 rpm, 25°C, 10 minutes). 100 µL of the supernatant was added to a 96-well plate, and the absorbance at 490 nm was measured using a plate reader ("Synergy H1" manufactured by BioTek).

The results are shown in FIG. 8. In FIG. 8, "mEG₂₄_1" and "CEL-C=CH" in the graph "6.3 (+)" on the left represent the results of the above test. The test was performed in three sets of three experiments, and the absorbance was determined as the grand mean of the mean values of the respective sets.

Incidentally, as a control, the reactions 1 to 3 were performed in the same manner as above, except that in the antigen-antibody reaction of the primary antibody described above, 100 µL of a 3.3 mass% BSA/PBS (pH 7.4) solution containing no 6.3 was added instead of 100 µL of the primary antibody solution. The results are shown in the graph "6.3 (-)" on the right in FIG. 8.

In addition, the reactions 1 to 3 were performed in the same manner as above, except that in the antigen-antibody reaction of the primary antibody described above, 10 µL of PBS containing no cellulose oligomer assembly was added instead of 10 µL of the cellulose dispersion. The results are shown as "(-)" in the graphs "6.3 (+)" and "6.3 (-)" in FIG. 8.

As shown in FIG. 8, with the cellulose oligomer assembly CEL-C=CH according to a comparative example, the absorbance was almost equivalent to that of "(-)" with no cellulose oligomer, and the anti-PEG antibody 6.3 did not bind. In contrast, with the cellulose oligomer assembly according to an example (mEG₂₄_1), the absorbance was significantly higher than that of "(-)", indicating the binding of the anti-PEG antibody 6.3. Therefore, with the cellulose oligomer assembly according to an example, backbone-selective anti-PEG antibodies can be detected.

Incidentally, with respect to the various numerical ranges described herein, the upper and lower limits thereof can be arbitrarily combined, and all such combinations are incorporated herein as preferred numerical ranges. In addition, the description of a numerical range "X to Y" means X or more and Y or less.

Some embodiments of the invention have been described above. However, these embodiments are presented as examples and not intended to limit the scope of the invention. These embodiments can be practiced in other various modes, and, without departing from the gist of the invention, various omissions, substitutions, and changes can be made thereto. These embodiments, as well as omissions, substitutions, and changes thereto, for example, fall within the scope and gist of the invention, and also fall within the scope of the claimed invention and its equivalents.

## Claims

1. An anti-PEG antibody-binding material comprising a cellulose oligomer represented by general formula (1): 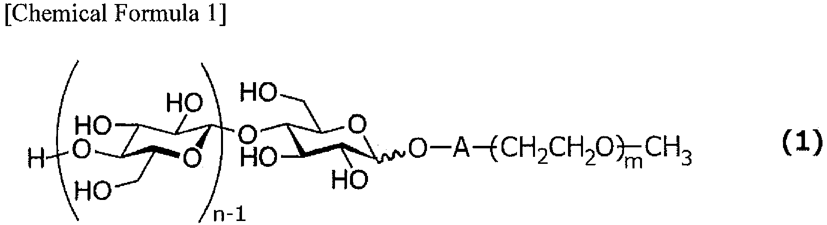 wherein n is the average degree of polymerization and represents a number of 6 to 16, m is the average degree of polymerization and represents a number of 3 or more, and A represents a divalent organic group that connects the oxygen atom bonded to the anomeric carbon of the reducing end of the cellulose oligomer and a polyethylene glycol chain and that has 1 to 24 carbon atoms and contains at least one heteroatom selected from the group consisting of a nitrogen atom, an oxygen atom, and a sulfur atom.

2. The anti-PEG antibody-binding material according to claim 1, wherein A in the formula (1) represents a divalent organic group having 2 to 10 carbon atoms and containing at least one member selected from the group consisting of a triazole group, an amide group, and a thioether group.

3. The anti-PEG antibody-binding material according to claim 1, wherein A in the formula (1) represents 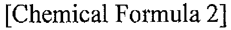 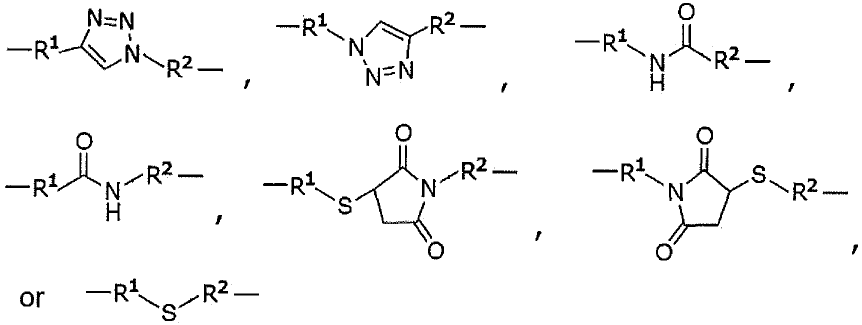 wherein R¹ represents a hydrocarbon group having 1 to 10 carbon atoms, and R² represents a single bond or a hydrocarbon group having 1 to 10 carbon atoms.

4. The anti-PEG antibody-binding material according to claim 1, wherein m in the formula (1) represents a number of 10 or more.

5. A biosensor comprising the anti-PEG antibody-binding material according to any one of claims 1 to 4.

6. An adsorbent comprising the anti-PEG antibody-binding material according to any one of claims 1 to 4.

7. A detection method for an anti-PEG antibody, comprising:
incubating, under conditions that allow binding to an anti-PEG antibody, a cellulose oligomer represented by general formula (1) with a specimen suspected of containing the anti-PEG antibody: 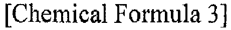 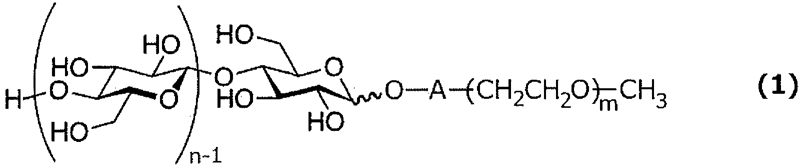 wherein n is the average degree of polymerization and represents a number of 6 to 16, m is the average degree of polymerization and represents a number of 3 or more, and A represents a divalent organic group that connects the oxygen atom bonded to the anomeric carbon of the reducing end of the cellulose oligomer and a polyethylene glycol chain and that has 1 to 24 carbon atoms and contains at least one heteroatom selected from the group consisting of a nitrogen atom, an oxygen atom, and a sulfur atom;
incubating, under conditions that allow binding of a secondary antibody to the anti-PEG antibody, the cellulose oligomer after incubation with the specimen with a sample containing the secondary antibody; and
detecting the anti-PEG antibody contained in the specimen using the cellulose oligomer after incubation with the sample, with the secondary antibody serving as a probe.
